# EUROPEAN PATENT APPLICATION

(11) **EP 1 338 605 A1**
(43) Date of publication of application: **27.08.2003**
(21) Application number: 01974349.1
(22) Date of filing: 11.10.2001
(51) Int. Cl.: C07K 5/08, A61K 38/06, A61P 25/00

(54) **N-ALKYLGLYCINE TRIMERES CAPABLE OF BLOCKING THE RESPONSE TO CHEMICAL SUBSTANCES, HEAT STIMULI OR MEDIATORS OF NEURONAL RECEPTOR INFLAMMATION AND COMPOSITIONS CONTAINING SAID TRIMERES**

(30) Priority: 11.10.2000 ES 200002458
(71) Applicant: Diverdrugs, S.L., 08902 Barcelona (ES)
(72) Inventor: FERRER MONTIEL, Antonio, E-08902 Barcelona (ES); PLANELLS CASES, Rosa, E-08902 Barcelona (ES); GARCIA MARTINEZ, Carolina, E-08902 Barcelona (ES); GONZALEZ ROS, José Miguel, E-08902 Barcelona (ES); MERINO FERNANDEZ, Jaime M., E-08902 Barcelona (ES); BEIMONTE MARTINEZ, Carlos, E-08902 Barcelona (ES); GALLAR MARTINEZ, Juana, E-08902 Barcelona (ES); PEREZ PAYA, Enrique, E-08902 Barcelona (ES); SANCHEZ BAEZA, Francisco, E-08902 Barcelona (ES); HUMET, Marc, E-08902 Barcelona (ES); MESSEGUER PEYPOCH, Angel, E-08902 Barcelona (ES)
(74) Representative: Maldonado Jordan, Julia
(86) International application number: ES0100384
(87) International publication number: WO02030956

(57) **Abstract**

The invention relates to N-alkylglycine trimeres (I), wherein R₁ represents 2-(2,4-dichlorophenyl)ethyl, 3-methylbutyl, 2-(methylcarbonylamino)ethyl, 2-(N-imidazolyl)ethyl or 3-(N,N-dimethylamino)propyl; R₂ represents 2-(2,4-dichlorophenyl)ethyl, 2-(4-methoxyphenyl)ethyl, 3,3-diphenylpropyl or 3-(N,N-diethylamino)propyl; and R₃ represents N,N-diethylaminopropyl, 3,3-diphenyl-propyl or 2-[2-(N-methyl)pyrrolidinyl]ethyl. Said trimeres are capable of blocking the ionic channels that are activated by exogenous chemical substances, heat stimuli or inflammation mediators, such as nociceptors VR1 and glutamate ionotropic receptors and are useful for treating disorders mediated by the activity of said receptors.

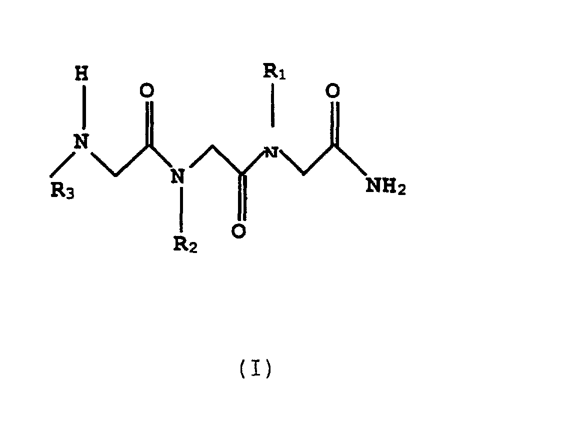

## Description

### SCOPE OF THE INVENTION

This invention refers to N-alkylglycine trimeres capable of blocking the response to chemical substances or heat stimuli or mediators of neuronal receptor inflammation, for example, the VR1 nociceptors and/or the ionotropic L-glutamate receptors, to compositions containing said trimeres and to their employment in the treatment of illnesses or disorders mediated by the activity of said receptors.

### BACKGROUND OF THE INVENTION

Pain represents a serious social and economic problem. It is calculated that more than 2 million people are daily incapacitated due to suffering transitory or chronic painful sensations. Clear examples are represented by the algesia experimented by patients suffering from cancer, migraine, arthritis, burns, and persons who have suffered accidents and surgical operations. In spite of the seriousness of the problem, the pharmacological arsenal for fighting, preventing and/or decreasing their symptoms and progress is surprisingly limited, due in part, to the lack of specific therapeutic targets on which to act.

The sensation of pain initiates when the peripherical terminals of a group of sensorial neurons, known as nociceptor neurons, are activated by noxious stimuli of chemical, mechanical or thermal nature (1,2) [see section relative to BIBLIOGRAPHY]. The nociceptor neurons transmit information relative to the tissular damage to the pain sensation processing centres in the spinal cord and the brain.

Though the precise biological pain transmission mechanisms are not clearly established, the cloning and characterisation of a capsaicin-activated membrane receptor (from here onwards identified as "VR1 nociceptor") have been recently described, which receptor is directly involved in pain pathways (3-5). The VR1 nociceptor is specifically expressed in sensorial neurones and mediates the response to noxious stimuli of chemical and thermal nature (3, 4, 6). This nociceptor is a ligand-activated ionic channel and is characterised in that it possesses a high calcium permeability (5,7). The high calcium permeability of the VR1 nociceptor is consistent with the neurodegeneration observed by the prolonged activation of nociceptor neurons, for example, under chronic pain conditions (1, 5, 6). Consequently, a strategy for decreasing the transmission and sensation of peripherical pain is to control the ionic channels involved in the chemical response of the nociceptors by means of developing specific and powerful antagonists.

In addition to the VR1 nociceptor, a description has also been made in relation to the participation of the L-glutamate excitatory neurotransmitter in pain transmission (8-10). The glutamate activates the membrane receptors that have an ionic channel activity (ionotropic receptors) or that transduce the signal through G proteins (metabotropic receptors) (11). The ionotropic glutamate receptors have been involved in the glutamatergic nociception due to its Ca²⁺ ions permeability (10, 11). The proposed molecular mechanism indicates that high and chronic glutamate levels cause a prolonged activation (hyperactivation) of the ionotropic receptors that load the sensorial neurones with Ca²⁺ ions, triggering off the massive and excessive activation of intracellular cascades that lead to pain transmission (2, 8). In fact, it has been described that the antagonists of these receptors are capable of producing analgesia (12-17). From what is expressed it can be deduced that a strategy to prevent or decrease the algesia is to control the functional activity of the glutamate ionotropic receptors, specifically, those present in the nociceptor neurons.

Consequently, it seems that the VR1 nociceptor and the L-glutamate ionotropic receptors are important therapeutic targets for efficiently controlling peripherical pain.

In spite of the advance that has been made in the past few years, powerful, selective and toxicity-free neuronal receptor inhibitors have not yet been developed. Great part of the effort made up to the moment has been carried out in the development of opioids that recognise opioid receptors of the central nervous system (1, 2). These molecules, though powerful analgesics, present important secondary effects, such as addiction, tolerance, cognitive anomalies, etc., that limit their clinical use (17, 18). An important effort has also been made to develop glutamate and/or glycine competitive and non competitive antagonists [a coagonist that participates in the activation of the NMDA type of glutamate (N-methyl-D-aspartate)]. These inhibitors have proved to be efficient and powerful, mitigating the sensation of pain, but have presented a restricted clinical use, once again, due to the secondary effects presented (10). The main disadvantage of using competitive and non competitive antagonists is that these molecules interact with their receptors inhibiting unspecifically the neurotransmission and affecting both the pathological activity of the glutamate and its physiological activity (10). A strategy for overcoming this therapeutic obstacle would be to use non competitive and/or acompetitive antagonists that preferably join the agonist-receptor complex. The most important advantage of using this type of antagonists is that these agents mainly act on the hyperactivated receptors (pathological receptors), showing a marginal interaction on receptors that perform in rapid excitatory neurotransmission processes (physiological receptors) (10). This activity that is preferred over the "pathological" receptors means that these types of antagonists are valued as promising therapeutic agents to prevent the transmission of pain (10-16), Molecules such as phencyclidine and the dizolcypine are powerful acompetitive antagonists of the NMDA receptor that act as efficient *in vitro* neuroprotectors (10, 14-16). However, their clinical use is questioned due to the psychotomimetic effects (10).

### SUMMARY OF THE INVENTION

The invention faces the problem of searching for new compounds capable of decreasing or treating peripherical pain sensation and that totally or partly overcome the previously indicated disadvantages.

The solution provided by this invention is based on the development of N-alkylglycine trimeres that are capable of blocking the ionic channels activated by exogenous chemical substances, heat stimuli or inflammation mediators such as VR1 nociceptors and the L-glutamate ionotropic receptors, and are useful for treating disorders mediated by the activity of said receptors.

The capability of said N-alkylglycine trimeres for blocking the VR1 nociceptors and NMDA type L-glutamate ionotropic receptors, has been made obvious by means of the tests described in Example 1.2, whilst their analgesic activity has been verified by means of the test described in Example 1.3.

Consequently, an object of this invention is constituted by N-alkylglycine trimeres capable of blocking the response to chemical substances, heat stimuli or mediators of nociceptor and L-glutamate ionotropic receptors inflammation, blocking the ionic channels of said receptors.

An additional object of this invention is constituted by a composition that comprises at least one of said N-alkylglycine trimeres, such as a pharmaceutical composition or a cosmetic composition.

The employment of said N-alkylglycine trimeres in the elaboration of a medicine for the treatment of disorders mediated by the activity of said receptors, constitutes another additional object of this invention.

### DETAILED DESCRIPTION OF THE INVENTION

The invention provides general formula (I) N-alkylglycine trimeres 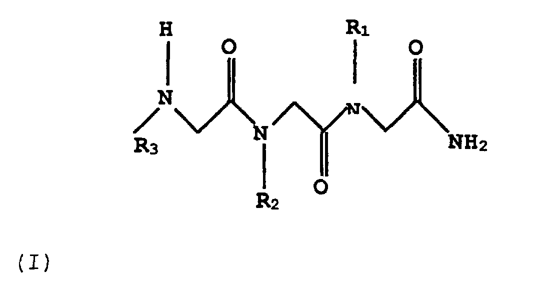 wherein
R₁ is chosen fromamongst 2-(2,4-dichlorophenyl)ethyl, 3-methyl-butyl, 2-(methylcarbonylamino)ethyl, 2-(N-imidazolyl)ethyl and 3-(N,N-dimethylamino)propyl;
R₂ is chosen fromamongst 2-(2,4-dichlorophenyl)ethyl, 2-(4-methoxyphenyl)ethyl, 3,3-diphenylpropyl and 3-(N,N-diethylamino)propyl; and
R₃ is chosen fromamongst N,N-diethylaminopropyl, 3,3-diphenylpropyl and 2-[2-(N-methyl)pyrrolidinyl]ethyl,
their stereoisomeric forms and mixtures, racemic or non racemic, of the same, and
their pharmaceutically acceptable salts.

Some of the formula (I) N-alkylglycine trimeres may have one or more chiral centres. Consequently, said formula (I) compounds may exist under any of their stereoisomeric forms (enantiomers or diastereoisomers) or in mixtures, racemic or non racemic, of the same, all of which fall within the scope of the present invention. Illustrative examples of formula (I) N-alkylglycine trimeres that contain chiral centres include those that contain groups 2-[2-(N-methyl)pyrrolidinyl]ethyl[(R,S)-2-[2-(N methyl)pyrrolidinyl]ethyl, (R)-2-[2-(N-methyl)pyrrolidinyl]ethyl or (S)-2-[2-(N-methyl)pyrrolidinyl]ethyl].

In a particular embodiment, the invention provides a formula (I) N-alkylglycine trimere that also contains a reversible modification with the purpose of increasing its bioavailability and ease of passage of the hematoencephalic barrier and epithelial tissue.

Illustrative examples of formula (I) N-alkylglycine trimeres provided by this invention are gathered in Table 1 [Example 1.1].

Within the scope of this invention are to be found the pharmaceutically acceptable salts of the formula (I) N-alkylglycine trimeres provided by this invention. The term "pharmaceutically acceptable salts" includes the salts normally used to form metallic salts or salts with acid additive. The nature of the salt is not critical, provided it is pharmaceutically acceptable. The pharmaceutically acceptable salts of formula (I) N-alkylglycine trimeres can be obtained as from acids, organic or inorganic. Said salts can be obtained by conventional methods, well known to the experts in the art, making the appropriate acid react with the formula (I) N-alkylglycine trimere.

Formula (I) N-alkylglycine trimeres can be obtained by conventional methods, for example, by means of copulation and amidation reactions among the glycine derivates that constitute the structural components of said trimeres. In turn, the glycine derivates can be obtained by means of conventional amino acid modification methods. The stereoisomeric forms of the formula (I) N-alkylglycine trimeres can be synthetisised as from the corresponding enantiomers or racemic or non racemic mixtures of the raw products. When parting from enantiomer mixtures, the stereoisomers obtained can be separated by means of conventional stereoisomeric resolution methods (enantiomers and diastereoisomers), for example, fractionized crystallisation, chromatography or salts formation.

Diverse tests have clearly shown that formula (I) N-alkylglycine trimeres are capable of blocking the ionic channel activity that is characteristic of the VR1 nociceptor and of the L-glutamate ionotropic receptors, preferably, of the NMDA type. Moreover, it has been observed that the formula (I) N-alkylglycine trimeres tested do not behave as competitive antagonists of the capsaicin ligand.

The capability of formula (I) N-alkylglycine trimeres for blocking the VR1 nociceptor ionic channel expressed in *Xenopus laevis* ovocites, as well as for blocking the ionic channel of the L-glutamate ionotropic receptors, in particular of the NMDA type, expressed in *X. laevis,* can be proved by means of a test that evaluates the efficiency and power of said N-alkylglycine trimeres blocking the ionic current activated by agonist in *X. laevis* ovocites that express said neuronal receptors [see Example 1.2]. These biological tests have the advantage that they permit the search of antagonists in functionally active receptors, increasing the power of their use *in vivo.*

The capability of formula (I) N-alkylglycine trimeres to evaluate its analgesic efficiency can be demonstrated by means of a test that consists in determining the effect of said N-alkylglycine trimeres on the electro-physiological response of the nociceptor sensorial nervous fibres that innervate the cornea of an anaesthetised cat, such as has been described in detail in Belmonte et al. (6). Briefly, the method consists of the dissection of the ciliary nerves that innervate the cornea with fine nervous fillets until one single afferent unit is located and identified by electric stimulation. The isolated fibre is subjected to electro-physiological studies that comprise the determination of the site and extension of the receptor field, the measure of the conduction rate, and the exploration of its chemo-sensibility to capsaicin, and the thermo-sensibility and mechano-sensibility threshold. Once all these parameters are determined and the afferent sensorial fibres electro-physiologically characterised, their modulation by the different antagonists of the VR1 nociceptor is studied [See Example 1.3].

Given the central role of the VR1 nociceptor in the integration of the pain pathways, a consequence of the blocker activity of said receptor by the action of the formula (I) N-alkylglycine trimeres is the potential development of a new family of analgesics. Moreover, the results obtained seem to indicate that the formula (I) N-alkylglycine trimeres, due to the blocker power of the VR1 nociceptor they manifest, are firm candidates for constituting a new generation of analgesics.

The formula (I) N-alkylglycine trimeres are useful as VR1 nociceptors ionic channel blockers and of the L-glutamate ionotropic receptors *per se,* due to which said N-alkylglycine trimeres are appropriate for the treatment of illnesses and pathological disorders mediated by the activity or function of the VR1 nociceptor ionic channels and/or of the L-glutamate ionotropic receptors, especially of the NMDA type, for example, the sensation of pain, in response to diverse noxious stimuli (mechanical, chemical and thermal).

Formula (I) N-alkylglycine trimeres may form part of diverse types of compositions for their application in the body of a mammal, preferably human beings. In this sense, the invention provides a composition that comprises at least one formula (I) N-alkylglycine trimere. In a particular embodiment, said composition is a pharmaceutical composition, whilst in another particular embodiment, said composition is a cosmetic composition.

The pharmaceutical composition provided by this invention comprises a therapeutically efficient amount of, at least one formula (I) N-alkylglycine trimere together with at least, one pharmaceutically acceptable excipient.

Formula (I) N-alkylgycine trimeres may be administered by any means that produce contact of the N-alkylglycine trimere with its site of action on the body of a mammal, preferably a human being.

The amount of therapeutically efficient formula (I) N-alkylglycine trimere that must be administered, as well as its dose for treating a pathological condition with the formula (I) N-alkylglycine trimere or with pharmaceutical compositions of the invention, shall depend on numerous factors, including age, condition of the patient, severity of the illness or disorder, route and frequency of the administration and the particular formula (I) N-alkylglycine trimere to be used.

The pharmaceutical composition provided by this invention can be presented under any form of administration, for example, solid or liquid, and may be administered by any appropriate manner, for example, orally, parentherally, rectal or topic, for which it shall include the pharmaceutically acceptable excipients necessary for the formulation of the desired form of administration. A revision of the different pharmaceutical forms of administration of medicines and of the excipients, necessary for the obtention of the same, may for example be found in the "Tratado de Farmacia Galenica", C. Fauli i Trillo, 1993, Luzán 5, S.A. Ediciones, Madrid.

Consequently, an additional object of this invention is constituted by the employment of a formula (I) N-alkylglycine trimere in the elaboration of a medicine for attenuating the nervous activity of primary sensorial neurons involved in the sensations of pain evoked by the application of exogenous chemical substances or by heat stimuli or by the endogenous liberation of chemical substances by inflamed tissues, or in the elaboration of a medicine that inhibits the ionic channels activated by exogenous chemical substances or by heat stimuli or by inflammation mediators that lead to the sensation of pain.

More specifically, the invention refers to the use of a formula (I) N-alkylglycine trimere in the elaboration of a medicine for the treatment of pathological illnesses and disorders mediated by the activity of the VR1 nociceptor ionic channels and L-glutamate ionotropic receptors, preferably of the NMDA type, for example, the sensation of pain in response to a noxious stimuli.

The invention additionally provides a method for the treatment of a patient with pathological illnesses and disorders mediated by the activity of the nociceptor ionic channels, for example, the sensation of pain mediated by the VR1 nociceptor and the L-glutamate ionotropic receptors in response to diverse noxious stimuli, for example, mechanical, chemical and thermal, that comprise the administration to said patient suffering from said pathological illness or disorder, a therapeutically efficient amount of formula (I) N-alkylglycine trimere, preferably in the form of a pharmaceutical composition containing it.

The cosmetic composition provided by this invention comprises a cosmetically efficient amount of at least, one formula (I) N-alkylglycine trimere together with at least, one cosmetically acceptable excipient or adjuvant.

Formula (I) N-alkylglycine trimeres may be administered in cosmetic compositions in order to calm, reduce, attenuate or relieve cutaneous pain or irritation produced by heat stimuli, for example after exposure to the sun; mechanical, for example, depilations, shaving; or slightly aggressive chemicals. The cosmetic composition of the invention may be presented under any appropriate form that permits the contact of the N-alkylglycine trimere with the site of action of the same on the body of the mammal on which it is applied.

The amount of formula (I) N-alkylglycine trimere to be administered shall depend on numerous factors, among which are to be found the degree of pain or irritation produced by the heat, mechanical or chemical stimuli, and of the N-alkylglycine trimere to be used.

The cosmetic composition provided by this invention may be presented under any administrational form, for example, solid or liquid and may be administered by any appropriate path, preferably topic, for which it shall include the cosmetically acceptable excipients or adjuvants that are appropriate for the form of presentation of the cosmetic composition. In a particular embodiment, the cosmetic composition provided by the invention is an after-sun product, for example, a cream, ointment, emulsion or "aftersun" lotion, appropriate for calming, reducing, attenuating or relieving the discomforts produced by sunburn due to exposure to the sun. In another particular embodiment, the cosmetic composition provided by the invention is an after-shave product, for example, a cream, an after-shaving balsam, emulsion or lotion, appropriate for calming, reducing, attenuating or relieving cutaneous pain or irritation produced by mechanical stimuli (shaving). In another particular embodiment, the cosmetic composition provided by the invention is an after-depilation product, appropriate for calming, reducing, attenuating or relieving cutaneous pain or irritation produced by the depilation. A revision of the different forms of presentation of cosmetic compositions and of the necessary excipients and adjuvants for their obtention may for example be found in "Cosmetologia Teórico-práctica", Prof. A. Del Pozo, published by the General Official Pharmacists College Board, 3^{rd} Issue, 1985.

In consequence, an additional object of this invention is constituted by the use of a formula (I) N-alkylglycine trimere in the elaboration of a cosmetic composition, appropriate for calming, reducing, attenuating or relieving cutaneous pain or irritation produced by heat, mechanical or chemical stimuli.

The invention moreover provides, a cosmetic method for calming, reducing, attenuating or relieving cutaneous pain or irritation produced by heat, mechanical or chemical stimuli in a mammal, preferably a human being, that comprises the administration to said mammal, of an efficient amount of formula (I) N-alkylglycine trimere, preferably under the form of a cosmetic composition that contains it.

On the other hand, an essential requirement for identifying bioactive molecules is to perform a test that permits the determination of its biological activity on the therapeutic targets. The inventors have developed a biological test that permits the evaluation of the power of molecules that block the ionic current activated by agonist in *X. laevis* ovocites that express neuronal receptors, such as VR1 nociceptor and the L-glutamate ionotropic receptors (19, 20). An important advantage of this biological test is that it permits the search for antagonists in functionally active receptors, increasing the power of its use *in vivo.*

The receptor heterologeous expression methods *in X. laevis* ovocites have been described in detail by Ferrer-Montiel and Montal (19).

Consequently, the invention also provides, in addition, a biological test to identify antagonists of the VR1 nociceptor and of the glutamatergic receptors, appropriate as high performance screening test for screening combining chemo-libraries of varied chemical diversity, as well as of isolated molecules.

This test comprises the contacting of *X. laevis* ovocites that express the VR1 nociceptor or glutamatergic receptors with an agonist (ligand) for said VR1 nociceptor, for example, capsaicin or L-glutamate, in the presence or absence of the compound to be tested, maintaining the transmembrane voltage constant at -80mV, and to detect the inhibiting activity of said compound to be tested by measuring the ionic current activated by the agonist in the presence and absence of the compound to be tested. The power and efficiency of the inhibiting activity of the compound to be tested can be determined by obtaining the dose-response curves. For this, the magnitude of the ionic current blockage activated by the *X. laevis* ovocite agonist that express the receptors in the presence of growing concentrations of the compound to be tested, is examined. The ionic current intensities ratio, in the presence and absence of the compound to be tested, allows to obtain the dose-response curves (19, 20). These graphs meet logarithmic functions to determine the maximum blockage (power) and the antagonist concentration that is produced by half the maximum blockage [IC₅₀ efficiency).

The following examples serve to illustrate the nature of the present invention and shall not be considered in its limitative sense. Example 1.1 describes the synthesis of some formula (I) N-alkylglycine trimeres, and Examples 1.2 and 1.3 illustrate the biological and analgesic activity of these compounds.

### EXAMPLE 1

### N-alkylglycine trimeres

### 1.1 N-alkylglycine trimeres synthesis

The N-alkylglycine trimeres identified in Table 1 were synthesised by means of conventional peptide synthesis methods in solid phase (21) the trimeres were purified by means of high resolution liquid chromatography.

**Table 1**

| **Formula (I) N-alkylglycine trimeres** | |
|---|---|
| [1] | [N-[(R,S)-2-[2-(N-methyl)pyrrolidinyl]ethyl]glycyl]-[N-(4-methoxyphenethyl)glycyl]-N-(3-methylbutyl)glycinamide; |
| [1a] | [N-[(R)-2-[2-(N-methyl)pyrrolidinyl]ethyl]glycyl]-[N-(4-methoxyphenethyl)glycyl]-N-(3-methylbutyl)glycinamide; |
| [1b] | [N-[(S)-2-[2-(N-methyl)pyrrolidinyl]ethyl]glycyl]-[N-(4-methoxyphenethyl)glycyl]-N-(3-methylbutyl)glycinamide; |
| [2] | [N-[(R,S)-2-[2- (N-methyl)pyrrolidinyl]ethyl]glycyl]-[N-(4-methoxyphenethyl)glycyl]-N-(2,4-dichlorophenethyl)glycinamide; |
| [2a] | [N-[(R)-2-[2-(N-methyl)pyrrolidinyl]ethyl]glycyl]-(N-(4-methoxyphenethyl)glycyl]-N-(2,4-dichlorophenethyl)glycinamide; |
| [2b] | [N- [(S)-2-[2-(N-methyl)pyrrolidinyl]ethyl]glycyl]-[N-(4-methoxyphenethyl)glycyl]-N-(2,4-dichlorophenethyl)glycinamide; |
| [3] | [N-[(R,S)-2-[2-(N-methyl)pyrrolidinyl]ethyl]glycyl]-[N-(2,4-dichlorophenethyl)glycyl]-N-(3-methylbutyl)glycinamide; |
| [3a] | [N-[(R)-2-[2-(N-methyl)pyrrolidinyl]ethyl]glycyl]-[N-(2,4-dichlorophenethyl)glycyl]-N-(3-methylbutyl)glycinamide; |
| [3b] | [N-[(S)-2-[2-(N-methyl)pyrrolidinyl]ethyl]glycyl]-[N-(2,4-dichlorophenethyl)glycyl]-N-(3-methylbutyl)glycinamide; |
| [4] | [N-[(R,S)-2-[2-(N-methyl)pyrrolidinyl]ethyl]glycyl]-[N-(2,4-dichlorophenethyl)glycyl]-N-(2,4-dichlorophenethyl)glycinamide; |
| [4a] | [N-[(R)-2-[2-(N-methyl)pyrrolidinyl]ethyl]glycyl]-[N-(2,4-dichlorophenethyl)glycyl]-N-(2,4-dichlorophenethyl)glycinamide; |
| [4b] | [N-[(S)-2-[2-(N-methyl)pyrrolidinyl]ethyl]glycyl]-[N-(2,4- |
| | dichlorophenethyl)glycyl]-N-(2,4-dichlorophenethyl)glycinamide; |
| [5] | [N-[3-(N,N-diethylamino)propyl]glycyl]-[N-(4-methoxyphenethyl)glicyl]-N-(3-methylbutyl)glycinamide; |
| [6] | [N-[3-(N,N-diethylamino)propyl]glycyl]-[N-(4-methoxyphenethyl)glicyl]-N-(2,4-dichlorophenethyl)glycinamide; |
| [7] | [N-[3-(N,N-diethylamino)propyl]glycyl]-[N-(2,4-dichlorophenethyl)glycyl]-N-(3-methylbutyl)glycinamide; |
| [8] | [N-[3-(N,N-diethylamino)propyl]glycyl]-[N-(2,4-dichlorophenethyl)glycyl]-N- (2,4-dichlorophenethyl)glycinamide; |
| [9] | [N-[3,3-diphenylpropyl)glycyl]-[N-[3-(N,N-diethylamino)propyl]glycyl]-[N-(2-methylcarbonylamino)ethyl]-N-(2,4-dichlorophenethyl)glycinamide; |
| [10] | [N-(3,3-diphenylpropyl)glycyl]-[N-[3-(N,N-diethylamino)propyl]glycyl]-[N-[2-(2-pyridyl)ethyl]glycinamide; |
| [11] | [N-(3,3-diphenylpropyl)glycyl]-[N-[3-(N,N-diethylamino)propyl]glycyl]-[N-[2-(N-imidazolyl)ethyl]glycinamide; |
| [12] | [N-(3,3-diphenylpropyl)glycyl]-[N-[3-(N,N-diethylamino)propyl]glycyl]-[N-[3-(N,N-dimethylamino)propyl]glycinamide; |
| [13] | [N-(3,3-diphenylpropyl)glycyl]-[N-(3,3-diphenylpropyl)glycyl]-N-[2-(methylcabonylamino)ethyl]glycinamide; |
| [14] | [N-(3,3-diphenylpropyl)glycyl]-[N-[3,3-diphenylpropyl)glycyl]-N-[2-(2-pyridyl)ethyl]glycinamide; |
| [15] | [N-(3,3-diphenylpropyl)glycyl]-[N-[3,3- |
| | diphenylpropyl)glycyl]-N-[2-(N-imidazolyl)ethyl]glycinamide; |
| [16] | [N-(3,3-diphenylpropyl)glycyl]-[N-[3,3-diphenylpropyl)glycyl] -N-[3-(N,N-dimethylamino)propyl]glycinamide; |
| [17] | [N-[(R,S)-2-[2-(N-methyl)pyrrolidinyl]ethyl]ghlycyl]-[N-[3-(N,N-diethylamino)propyl]glycyl]-N-[2-(methylcarbonylamino)ethyl]glycinamide; |
| [17a] | [N-[(R)-2-[2-(N-methyl)pyrrolidinyl]ethyl]glycyl]-[N-[3-(N,N-diethylamino)propyl]glycyl]-N-[2-(methylcarbonylamino)ethyl]glycinamide; |
| [17b] | [N-[(S)-2-[2-(N-methyl)pyrrolidinyl]ethyl]glycyl]-[N-[3-(N,N-diethylamino)propyl]glycyl]-N-[2-(methylcarbonylamino)ethyl]glycinamide; |
| [18] | [N-[(R,S)-2-[2-(N-methyl)pyrrolidinyl]ethyl]glycyl]-[N-[3-(N,N-diethylamino)propyl]glycyl]-N-[2-(2-pyridyl)ethyl]glycinamide; |
| [18a] | [N-[(R)-2-[2-(N-methyl)pyrrolidinyl]ethyl]glycyl]-[N-[3-(N,N-diethylamino)propyl]glycyl]-N-[2-(2-pyridyl)ethyl]glycinamide; |
| [18b] | [N-[(S)-2-[2-(N-methyl)pyrrolidinyl]ethyl]glycyl]-[N-[3-(N,N-diethylamino)propyl]glycyl]-N-[2-(2-pyridyl)ethyl]glycinamide; |
| [19] | [N-[(R,S)-2-[2-(N-methyl)pyrrolidinyl]ethyl]glycyl]-[N-[3-(N,N-diethylamino)propyl]glycyl]-N-[2-(N-imidazolyl)ethyl]glycinamide; |
| [19a] | [N-[(R)-2-[2-(N- |
| | methyl)pyrrolidinyl]ethyl]glycyl]-[N-[3-(N,N-diethylamino)propyl]glycyl]-N-[2-(N-imidazolyl)ethyl]glycinamide; |
| [19b] | [N-[(S)-2-[2-(N-methyl)pyrrolidinyl]ethyl]glycyl]-[N-[3-(N,N-diethylamino)propyl]glycyl]-N-[2-(N-imidazolyl) ethyl] glycinamide; |
| [20] | [N-[(R,S)-2-[2-(N-methyl)pyrrolidinyl]ethyl]glycyl]-[N-[3-(N,N-diethylamino)propyl]glycyl]-N-[3-(N,N-dimethylamino)propyl]glycinamide; |
| [20a] | [N-[(R)-2-[2-(N-methyl)pyrrolidinyl]ethyl]glycyl]-[N-[3-(N,N-diethylamino)propyl]glycyl]-N-[3-(N,N-dimethylamino)propyl]glycinamide; |
| [20b] | [N-[(S)-2-[2-(N-methyl)pyrrolidinyl]ethyl]glycyl]-[N-[3-(N,N-diethylamino)propyl]glycyl]-N-[3-(N,N-dimethylamino)propyl]glycinamide; |
| [21] | [N-[(R,S)-2-[2-(N-methyl)pyrrolidinyl]ethyl]glycyl]-[N-(3,3-diphenylpropyl)glycyl]-N-[2-(methylcarbonylamino)ethyl]glycylamide; |
| [21a] | [N-[(R)-2-[2-(N-methyl)pyrrolidinyl]ethyl]glycyl]-[N-(3,3-diphenylpropyl)glycyl]-N-[2-(methylcarbonylamino)ethyl]glycylamide; |
| [21b] | [N-[(S)-2-[2-(N-methyl)pyrrolidinyl]ethyl]glycyl]-[N-(3,3-diphenylpropyl)glycyl]-N-[2-(methylcarbonylamino)ethyl]glycylamide; |
| [22] | [N-[(R,S)-2-[2-(N-methyl)pyrrolidinyl]ethyl]glycyl]-[N-(3,3-diphenylpropyl)glycyl]-N-[2-(2- |
| | pyridyl)ethyl]glycinamide; |
| [22a] | [N-[(R)-2-[2-(N-methyl)pyrrolidinyl]ethyl]glycyl]-[N-(3,3-diphenylpropyl)glycyl]-N-[2-(2-pyridyl)ethyl]glycinamide; |
| [22b] | [N-[(S)-2-[2-(N-methyl)pyrrolidinyl] ethyl]glycyl]-[N- (3,3-diphenylpropyl)glycyl]-N-[2- (2-pyridyl)ethyl]glycinamide; |
| [23] | [N-[(R,S)-2-[2-(N-methyl)pyrrolidinyl]ethyl]glycyl]-[N-(3,3-diphenylpropyl)glycyl]-N-[2-(N-imidazolyl)ethyl]glycinamide; |
| [23a] | [N-[(R)-2-[2-(N-methyl]pyrrolidinyl]ethyl]glycyl]-[N-(3,3-diphenylpropyl)glycyl]-N-[2-(N-imidazolyl)ethyl]glycinamide; |
| [23b] | [N-[(S)-2-[2-(N-methyl)pyrrolidinyl]ethyl]glycyl]-[N-(3,3-diphenylpropyl)glycyl]-N-[2-(N-imidazolyl)ethyl]glycinamide; ; |
| [24] | [N-[(R,S)-2-[2-(N-methyl)pyrrolidinyl]ethyl]glycyl]-[N-(3,3-diphenylpropyl)glycyl]-N-[3-(N,N-dimethylamino)propyl)glycinamide; |
| [24a] | [N- [(R)-2-[2-(N-methyl)pyrrolidinyl]ethyl]glycyl]-[N-(3,3-diphenylpropyl)glycyl]-N-[3-(N,N-dimethylamino)propyl)glycinamide; |
| [24b] | [N-[(S)-2-[2-(N-methyl)pyrrolidinyl]ethyl]glycyl]-[N-(3,3-diphenylpropyl)glycyl]-N-[3-(N,N-dimethylamino)propyl)glycinamide; |

### 1.2 Biological activity evaluation

### 1.2.1. Blocking of the VR1 nociceptor

In order to evaluate the biological activity of the N-alkylglycine trimeres obtained in Example 1.1 on VR1 nociceptors, a test was developed that evaluated the efficiency and power of said compounds that block the ionic current activated by agonist in *X. laevis* ovocites that express the VR1 nociceptor.

The heterologeous expression of receptors in *X.laevis* ovocites can be performed according to the procedure described by Ferrer-Montiel and Montal (19). Briefly, the *X. laevis* ovocites of adult frogs are gathered, manipulated, and injected with cDNA that codes the VR1 nociceptor (5, 19). The ionic currents activated by the capsaicin agonist are recorded with the maintenance of the constant voltage technique with two microelectrodes (two-microelectrode voltage clamp) (19, 20). The ovocites that express the VR1 nociceptor are transferred to the recording chamber and perfunded using an 8-output perfusion system. The capsaicin, both in the absence and in the presence of the N-alkylglycine trimeres to be tested, is dissolved in a Ringer buffer (Hepes 10 mM pH 7,4 NaC1 100 mM, BaCl₂ 2,0 mM, MgCl₂ 2,0 mM, KC1 3,0 mM) supplemented with 100 µM fluphenamic acid (19, 20). This buffered solution is used to minimize the contribution of the endogenous chloride ionic channel activated by calcium to the ionic current of the VR1 nociceptor (19, 20). The transmembrane voltage is kept constant at -80 mV, and the ionic currents are activated by applying pulses of the capsaicin solution (20 µM) in the presence or absence of growing concentrations of the N-alkylglycine trimeres to be tested. The inhibiting activity is detected by measuring the ionic current activated by the agonist in the presence and absence of the N-alkylglycine trimeres.

The power and efficiency of the inhibitory activity of N-alkylglycine trimeres is determined by obtaining the dose-response curves. For this, the magnitude of the ionic current blockage activated by the capsaicin in ovocites that express the VR1 nociceptor in the presence of growing N-alkylglycine trimere concentrations, is examined. The ionic current intensities ratio in the presence and absence of said N-alkylglycine trimeres is used to obtain the dose-response curves (19, 20). These graphs adjust to logarithmic functions for determining the maximum blockage (power) and the antagonist concentration that is produced by half the maximum blockage (IC₅₀ efficiency).

The results obtained showed that all the tested N-alkylglycine trimeres blocked the ionic channel activity, characteristic of the VR1 nociceptor expressed in frog *X. laevis* ovocites. The N-alkylglycine trimere concentrations that inhibited half the VR1 nociceptor response (IC₅₀) activated by capsaicin at a concentration of 20 µM were approximately 1 µM for the N-alkylglycine trimere identified as compound [4], of approximately 2 µM for the N-alkylglycine trimere identified as compound [8] and of approximately 30 µM for the N-alkylglycine trimere identified as compound [7].

On the other hand, due to the fact that some formula (I) N-alkylglycine trimeres may be presented as stereoisomers, the possibility of whether the blocker activity of the VR1 nociceptor of the racemic mixture preferably corresponded to one of the isomers, was evaluated. For this, the blocker activity of the VR1 nociceptor of enantiomers identified as [4a] and [4b] (see Table 1) was evaluated, according to the previously described protocol, and it was compared with the blocker activity of said racemic mixture receptor of said enantiomers. The results obtained were similar in both cases and showed that both enantiomers perform a similar blocker activity of the VR1 nociceptors.

Given the role of the nociceptors in the integration of pain pathways, a consequence of the blocker activity of these N-alkylglycine trimeres is their employment as analgesic. 1.2.2 Blocking of type NMDA glutamatergic receptor.

In order to evaluate the biological activity of the N-alkylglycine trimeres obtained in Example 1.1 on type NMDA glutamatergic receptors, a test was developed that evaluated the efficiency and power of said compounds blocking the ionic current activated by *X. laevis* ovocite agonist that express the type NMDA receptor, the VR1 nociceptor.

The heterologeous expression of receptors in *X. laevis* ovocites may be carried out according to the procedure described by Ferrer-Montiel and Montal (19). Briefly, the *X. laevis* ovocites of adult frogs are gathered, manipulated and injected with cRNA that codes the NR1 and NR2A subunities of the NMDA receptor (5, 20). The ionic currents activated by L-glutamate agonist in the presence of the glycine coagonist are recorded with the constant voltage maintenance technique with two microelectrodes (two-microelectrode voltage clamp) (19, 20). The ovocites that express the receptor are transferred to the recording chamber and perfunded using an 8-output perfusion system. The agonist and the coagonist, both in the absence and in the presence of the N-alkylglcycine trimeres to be tested, are dissolved in a Ringer buffer (Hepes 10 mM pH 7,4 NaC1 100 mM, Ba Cl₂, 2,0 mM, KC1 3,0 mM) supplemented with 100 µM fluphenamic acid (20). This buffered solution is used to minimize the contribution of the chloride endogenous ionic channel activated by calcium to the glutamatergic receptor ionic current (19, 20). The transmembrane voltage is kept constant at -80 mV and the ionic currents are activated by applying pulses of the L-glutamate/glycine solution (100 µM / 20 µM) in the presence or absence of growing concentrations of the N-alkylglycine trimeres to be tested. The inhibiting activity is detected by measuring the ionic current activated by the agonist in the presence and absence of the N-alkylglycine trimeres.

The power and efficiency of the N-alkylglycine trimeres inhibiting activity is determined by obtaining the dose-response curves. For this, the magnitude of the ionic current blockage activated by L-glutamate/glycine in ovocites that express the NMDA receptor in the presence of growing concentrations of the N-alkylglycine trimeres, is examined. The ionic current intensity ratios, in the presence and absence of said N-alkylglycine trimeres, is used to obtain the dose-response curves (19, 20). These graphs meet logarithmic functions to determine the maximum blockage (power) and the antagonist concentration that is produced by half the maximum blockage (IC₅₀ efficiency).

The results obtained showed that all the N-alkylglycine trimeres tested blocked the ionic channel activity, characteristic of the NMDA receptor expressed in frog *X. laevis* ovocites. The N-alkylglycine trimere concentrations that inhibited half the NMDA receptor response (IC₅₀) activated by L-glutamate/glycine at a 100 µM/20 µM concentration oscillated between 0,1 µM and 100 µM for the different N-alkylglycine trimeres tested (Table 1). Thus, for example, the IC₅₀ of the N-alkylglycine trimere identified as compound [9] is 1.0 µM, whilst for the N-alkylglycine trimere identified as compound [10] it is of approximately 2 µM, and of approximately 10 µM for the N-alkylglycine trimere identified as compound [11].

### 1.3 Evaluation of the analgesic activity

An investigation was performed on the attenuation of the N-alkylglycine trimeres indicated in Table 1, on the irritation produced by the topical application of capsaicin in the eyes of animals, an ocular pain model (22). The experimental paradigm compares the number of scratches after the topical administration of capsaicin at 0,01% in the eye of a mouse, in the absence and presence of the N-alkylglycine trimeres. After the administration of capsaicin, the number of times the mouse scratches its treated eye during 1 minute is counted. Subsequently, the affected eye is extensively rinsed with saline solution and 15 minutes after application, the compound to be tested is administered. After 5 minutes incubation, a 0,1% capsaicin solution containing the compound to be tested is administered. The number of times the animal scratches its eye during 1 minute is immediately counted. The analgesic capacity is obtained by comparing the number of scratches in the first application of capsaicin (without the compound to be tested) and the second application of the irritant (in the presence of the compound to be tested). A decrease of scratches in the second application of capsaicin is indicative of an analgesic activity of the N-alkylglycine trimere tested.

The results obtained with the N-alkylglycine trimeres identified as compounds [4] and [8] showed that compound [4] attenuated approximately 40% of the ocular irritation produced by capsaicin, whilst compound [8] reduced it by approximately 20%, what permits the affirmation that said compounds have analgesic activity tested in an animal pain model.

### BIBLIOGRAPHY

1. Fields, H.L. (1987) Pain (McGraw-Hill), New York.
2. Baranauskas, G. and Nistri, A. (1998) Sensitization of pain pathways in the spinal cord: cellular mechanisms. *Frog. Neurobiol. 54,* 349-365.
3. Liu, L. and Simon, S.A. (1994) A rapid capsaicin-activated current in rat trigeminal ganglion neurons. *Proc. Natl. Acad. Sci. USA 91,* 738-741
4. Szallasi, A. & Blumberg, P.M. (1996) Vanilloid receptors: new insights enhance potential as therapeutic target. *Pain 68,* 195-208.
5. Caterina, M.J. Schumacher, M.A., Tominaga, M., Rossen, T.A., Levine, J.D. and Julius, D. (1997) The capsaicin receptor: a heat-activated ion channel in the pain pathway. *Nature 389,* 816-824.
6. Belmonte, C., Gallar, J., Pozo, M.A. and Rebollo, I. (1991) Excitation by irritant chemical substances of sensory afferent units in the cat's cornea. J. *Physiol. (Lond)* 437, 709-725.
7. Garcia-Hirschfeld, J., López-Briones, L.G.,Belmonte, C. and Valdeolmillos, M. (1994) Intracellular free calcium responses to protons and capsaicin in cultured trigeminal neurons. *Neurosci. 67,* 235-243.
8. Sakurada, T., Wako, K., Sugiyama, A., Sakurada, C., Tan-No, K. and Kisara, K. (1998) Involvement of spinal NMDA receptors in capsaicin-induced nociception. *Pharmacol. Biochem. Behav. 59,* 339-345.
9. Omote, K., Kawamata, T., Kawamata, M. and Namiki, A. (1998) Formalin-induced release of excitatory amino acids in the skin of rat hindpaw. *Brain Res. 787,* 161-164.
10. Lipton, S.A. and Rosenburg, P.A. (1994). Excitatory amino acids as a final common pathway for neurologic disorders. *New Engl. J. Med. 330,* 613-622.
11. Nakanishi, S. and Masu, M. (1994) Molecular diversity and functions of glutamate receptors (1994) Annu. Rev. *Biophys. Biomol. Struc. 23,* 319-348.
12. Zahn, P.K, Umali, E. and Breannan, T.J. (1998) Intrathecal non-NMDA excitatory amino acid receptor antagonists inhibit pain behaviours in a rat model of postoperative pain. *Pain 74,* 213-223.
13. Nicolodi, M., Del Bianco, P.L. and Sicuteri, F. (1997) Modulation of excitatory amino acids pathway: a possible therapeutic approach to chronic daily headache associated with analgesic drug abuse. *Int. J. Clin. Pharmacol. Res. 17,* 97-100.
14. Wiesenfeld-Hallin, Z. (1998) Combined opioid-NMDA antagonist therapies. What advantages do they offer for the control of pain symdromes? *Drugs 55,* 1-4.
15. Davidson, E.M. and Carlton, S.M. (1998) Interplanar injection of dextrorphan, ketamine or memantine attenuates formalin-induced behaviours. *Brain Res. 785.* 136-142.
16. Eisenberf, E. and Pud, D. (1998) Can patients with chronic neuropathic pain be cured by acute administration of the NMDA receptor antagonist amantadine?. *Pain 74,* 337-339.
17. Karlsten, R. and Gordh, T. (1997) How do drugs relieve neurogenic pain? *Drugs Aging 11,* 398-412.
18. González, P., Cabello, P., Germany, A., Norris, B. and Contreras, E. (1997) Decrease of tolerance to, and physical dependence on morphine by, glutamate receptor antagonists. *Eur. J. Pharmacol.* 332, 257-262.
19. Ferrer-Montiel, A.V. and Montal, M. (1994). Structure-function relations in ligand-gated ion channels: Reconstitution in lipid bilayers and heterologous expression in *Xenopus* oocytes. *Methods: a Companion to Methods in Enzymology 6,* 60-69.
20. Ferrer-Montiel, A.V., Sun, W. and Montal, M. (1995). Molecular design of the NMDA receptor binding site for PCP and MK-801. *Proc. Natl. Acad. Sci. USA. 92,* 8021-8025.
21. Figliozzi, G.M.m, Goldsmith, R., Ng., S.C., Banville, S.C., Zuckermann, R.N. (1996) Synthesis of N-substituted glycine peptoid libraries. *Method Enzymol. 267,* 437-447.
22. González, G.G., Garcia de la Rubia, P., Gallar, J. and Belmonte, C. (1993) Reduction of capsaicin-induced ocular pain and neurogenic inflammation by calcium antagonists. *Investig. Ophtalmol. Visual Sci. 34,* 3329-3335.

## Claims

1. Use of a general formula (I) N-alkylglycine trimere 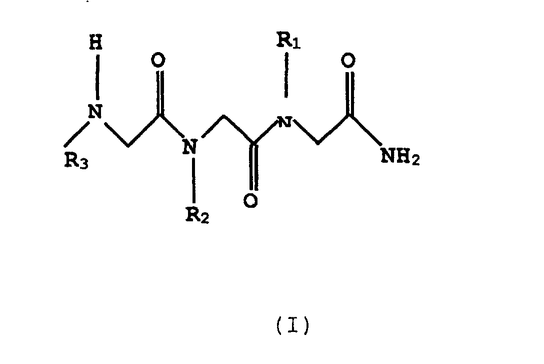 wherein
R₁ is chosen fromamongst 2-(2,4-dichlorophenyl)ethyl, 3-methylbutyl, 2-(methylcarbonylamino)ethyl, 2-(N-imidazolyl)ethyl and 3-(N,N-dimethylamino)propyl;
R₂ is chosen fromamongst 2-(2,4-dichlorophenyl)ethyl, 2-(4-methoxyphenyl)ethyl, 3,3-diphenylpropyl and 3-(N,N-diethylamino)propyl; and
R₃ is chosen fromamongst N,N-diethylaminopropyl, 3,3-diphenylpropyl and 2-[2-(N-methyl)pyrrolydinyl]ethyl,
their stereoisomeric forms and mixtures, racemic or non racemic of the same, and
their pharmaceutically acceptable salts,
in the elaboration of a medicine for the attenuation of nervous activity of primary sensorial neurons involved in the sensations of pain induced by the application of exogenous chemical substances or by heat stimuli or by the endogenous liberation of chemical substances by inflamed tissues.

2. Use of a general formula (I) N-alkylglycine trimere 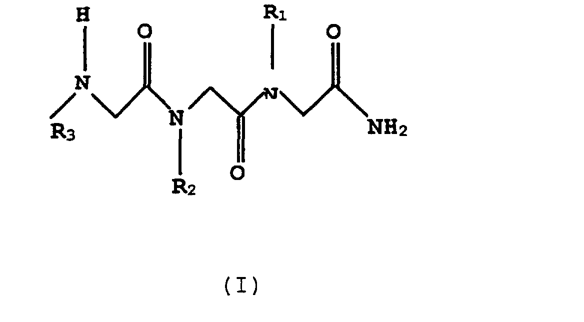 wherein
R₁ is chosen fromamongst 2-(2,4-dichlorophenyl)ethyl, 3-methylbutyl, 2-(methylcarbonylamino)ethyl, 2-(N-imidazolyl)ethyl and 3-(N,N-dimethylamino)propyl;
R₂ is chosen fromamongst 2-(2,4-dichlorophenyl)ethyl, 2-(4-methoxyphenyl)ethyl, 3,3-diphenylpropyl and 3-(N,N-diethylamino)propyl; and
R₃ is chosen fromamongst N,N-diethylaminopropyl, 3,3-diphenylpropyl and 2-[2-(N-methyl)pyrrolidinyl]ethyl,
their stereoisomeric forms and mixtures, racemic or non racemic of the same, and
their pharmaceutically acceptable salts,
in the elaboration of a medicine that inhibits the ionic channels activated by exogenous chemical substances or by heat stimuli or by inflammation mediators that lead to the sensation of pain

3. Use of a general formula (I) N-alkylglycine trimere. 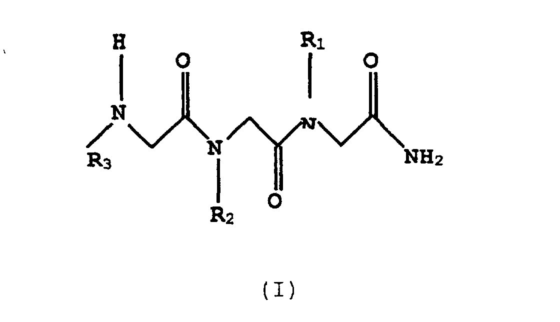 wherein
R₁ is chosen fromamongst 2-(2,4-dichlorophenyl)ethyl, 3-methylbutyl, 2-(methylcarbonylamino)ethyl, 2-(N-imidazolyl)ethyl and 3-(N,N-dimethylamino)propyl;
R₂ is chosen fromamongst 2-(2,4-dichlorophenyl)ethyl, 2-(4-methoxyphenyl)ethyl, 3,3-diphenylpropyl and 3-(N,N-diethylamino)propyl; and
R₃ is chosen fromamongst N,N-diethylaminopropyl, 3,3-diphenylpropyl and 2-[2-(N-methyl)pyrrolidinyl]ethyl,
their stereoisomeric forms and mixtures, racemic or non racemic, of the same, and
their pharmaceutically acceptable salts,
in the elaboration of a medicine for the treatment of pathological illnesses and disorders mediated by ionic channels activity of nociceptors VR1 and/or of glutamate ionotropic receptors.

4. Use of an N-alkylglycine trimere according to claim 3, in the elaboration of a medicine for the treatment of pain.

5. Use of an N-allkylglycine trimere according to claim 3, in the elaboration of a medicine for the treatment of heat hyperalgesia or heat sensibilization.

6. Use of an N-alquilglycine trimere according to claim 3, in the elaboration of a medicine for the treatment of neuropathic pain and neurogenic inflammation.

7. A pharmaceutical composition that comprises a therapeutically efficient amount of, at least, one N-alkylglycine trimere according to any of the previous claims, and, at least, one pharmaceutically acceptable excipient.

8. A dermo-cosmetic composition according to claims 1, 2 and 5, that comprises a cosmetically efficient amount of, at least, a general formula (I) N-alkylglycine trimere 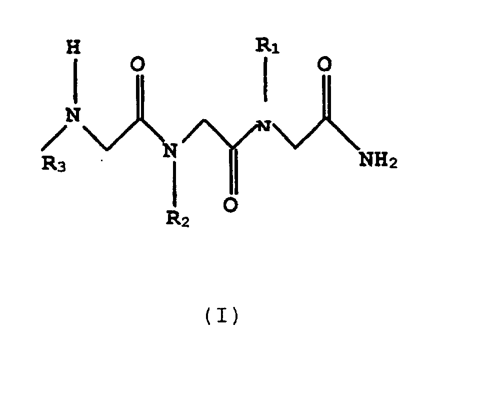 wherein
R₁ is chosen fromamongst 2-(2,4-dichlorophenyl)ethyl, 3-methylbutyl, 2-(methylcarbonylamino)ethyl, 2-(N-imidazolyl)ethyl and 3-(N,N-dimethylamino)propyl;
R₂ is chosen fromamongst 2-(2,4-dichlorophenyl) ethyl, 2-(4-methoxyphenyl)ethyl, 3,3-diphenylpropyl and 3-(N,N-diethylamino) propyl; and
R₃ is chosen fromamongst N,N-diethylaminopropyl, 3,3-diphenylpropyl and 2-[2-(N-methyl)pyrrolidinyl]ethyl,
their stereoisomeric forms and mixtures, racemic or non racemic, of the same, and
their cosmetically acceptable salts, and
at least, one cosmetically acceptable excipient or adjuvant.

9. Dermo-cosmetic composition according to claim 8, for skin treatment and care, selected from among an aftersun product, an after-shave product and an after-depilation product.

10. Use of a general formula (I) N-alkylglycine trimere 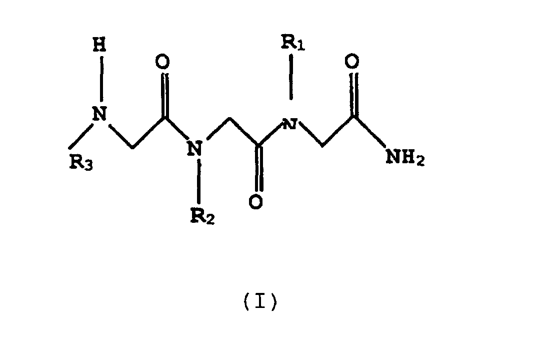 wherein
R₁ is chosen fromamongst 2-(2,4-dichlorophenyl)ethyl, 3-methylbutyl, 2-(methylcarbonylamino)ethyl, 2-(N-imidazolyl)ethyl and 3-(N,N-dimethylamino)propyl,
R₂ is chosen fromamongst 2-(2,4-dichlorophenyl)ethyl, 2-(4-methoxyphenyl)ethyl, 3,3-diphenylpropyl and 3-(N,N-diethylamino)propyl; and
R₃ is chosen fromamongst N,N-diethylaminopropyl, 3,3-diphenylpropyl, and 2-[2-(N-methyl)pyrrolidinyl]ethyl,
their stereoisomeric forms and mixtures, racemic or non racemic, of the same, and
their cosmetically acceptable salts,
according to claims 1, 2 and 5, in the elaboration of a dermo-cosmetic composition useful for calming, reducing, attenuating or relieving cutaneous pain or irritation produced by heat, mechanical or chemical stimuli or by heat hyperalgesia.

## Amended claims

### Amended claims under Art. 19.1 PCT

**1.** A general formula (I) N-alkylglycine trimer 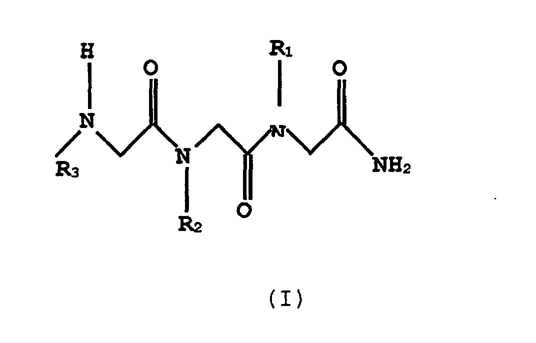 where
R₁ is selected from among 2-(2,4-dichlorophenyl)ethyl, 3-methylbutyl, 2-(methylcarbonylamine)ethyl, 2-(N-imidazolyl)ethyl and 3-(N,N-dimethylamine)propyl;
R₂ is selected from among 2-(2,4-cichlorophenyl)ethyl, 2-(4-methoxyphenyl)ethyl, 3,3-diphenylpropyl and 3-(N,N-diethylamine)propyl; and
R₃ is selected from among N,N-diethylaminopropyl, 3,3-diphenylpropyl and 2-[2-(N-methyl)pyrrolydyl]ethyl, and their pharmaceutically acceptable salts.

**2.** A general formula (I) N-alkylglycine trimer according to Claim 1, selected from among:
[1] [N-[2-[2-(N-methylpyrrolidinyl] ethyl]glycyl]-[N-(4-methoxyphenethyl)glycyl]-N-(3-methylbutyl)glycinamide
[2] : [N-[2-[2-(N-methylpyrrolidinyl] ethyl] glycyl] - [N- (4-methoxyphenethyl)glycyl]-N-(2,4-dichlorophenethyl) glycinamide
[3] : dichlorophenethyl)glycinamide [N-[(2-[2-(N-methylpyrrolidinyl] ethyl] glycyl] - [N- (2,4-dichlorophenethyl)glycyl]-N-(3-methylbutyl)glycinamide
[4] : [N-[2-[2-(N-methylpyrrolidinyl]ethyl]glycyl]-[N-(2,4-dichlorophenethyl)glycyl]-N-(2,4-dichlorophenethyl)glycinamide
[5] : [N-[3-(N,N-diethylamine)propyl]glycyl]-[N-(4-methoxyphenethyl)glycyl]-N-(3-methylbutyl)glycinamide
[6]: [N-[3-(N,N-diethylamine)propyl]glycyl]-[N-(4-methoxyphenethyl)glycyl]-N- (2,4-dichlorophenethyl)glycinamide
[7] : [N-[3-(N,N-diethylamine)propyl]glycyl]-[N-(2,4-dichlorophenethyl)glycyl]-N-(3-methylbutyl)glycinamide
[8] : [N-[3-(N,N-diethylamine)propyl]glycyl]-[N-(2,4-dichlorophenethyl)glycyl]-N-(2,4-dichlorophenethyl)glycinamide
[9]: [N-[3,3-diphenylpropyl)glycyl]-N-[3-(N,N-diethylamine)propyl]glycyl]-[N- (2-methylcarbonylamine)ethyl]-N-(2,4-dichlorophenethyl) glycinamide
[10] : [N-(3,3-diphenylpropyl)glycyl]-[N-[3-(N,N-diethylamine)propyl]glycyl]-[N-[2-(2-pyridyl)ethyl]glycinamide
[11] : [N-(3,3-diphenylpropyl)glycyl]-[N-[3-(N,N-diethylamine)propyl]glycyl]-[N-[2-(N-imidazolyl)ethyl]glycinamide
[12] [N-(3,3-diphenylpropyl)glycyl]-[N-[3-(N,N-diethylamine)propyl]glycyl]-[N-[3-(N,N-dimethylamine)propyl]glycinamide
[13]: [N-(3,3-diphenylpropyl)glycyl]-[N-(3,3-diphenylpropyl)glycyl]-N-[2-(methylcabonylamine)ethyl]glycinamide
[14]: [N-(3,3-diphenylpropyl)glycyl]-[N-[3,3-diphenylpropyl)glycyl]-N-[2-(2-pyridyl)ethyl]glycinamide
[15]: [N-(3,3-diphenylpropyl)glycyl]-[N-[3,3-diphenylpropyl)glycyl]-N-[2-(N-imidazolyl)ethyl]glycinamide
[16]: [N-(3,3-diphenylpropyl)glycyl]-[N-[3,3-diphenylpropyl)glycyl]-N-[3-(N,N-dimethylamine)propyl]glycinamide
[17]: [N-[2-[2-(N-methylpyrrolidinyl] ethyl]glycyl]-[N-[3-(N,N-diethylamine)propyl]glycyl]-N-[2-(methylcarbonylamine)ethyl]glycinamide
[18]: [N-[2-[2-(N-methylpyrrolidinyl] ethyl]glycyl]-[N-[3-(N,N-diethylamine)propyl]glycyl]-N-[2-(2-pyridyl)ethyl]glycinamide
[19]: [N-[2-[2-(N-methylpyrrolidinyl] ethyl]glycyl]-[N-[3-(N,N-diethylamine)propyl]glycyl]-N-[2-(N-imidazolyl)ethyl]glycinamide
[20]: [N-[2-[2-(N-methylpyrrolidinyl] ethyl]glycyl]-[N-[3-(N,N-diethylamine)propyl]glycyl]-N-[3-(N,N-dimethylamine)propyl]glycinamide
[21]: [N-[2-[2-(N-methylpyrrolidinyl] ethyl]glycyl]-[N-(3,3-diphenylpropyl)glycyl]-N-[2-(methylcarbonylamine)ethyl]glycylamide
[22] : [N-[2-[2-(N-methylpyrrolidinyl] ethyl]glycyl]-[N-(3,3-diphenylpropyl)glycyl]-N-[2-(2-pyridyl)ethyl]glycinamide
[23]: [N-[2-[2-(N-methylpyrrolidinyl] ethyl]glycyl]-[N-(3,3-diphenylpropyl)glycyl]-N-[2-(N-imidazolyl)ethyl]glycinamide
[24]: [N-[2-[2-(N-methylpyrrolidinyl] ethyl] glycyl] - [N-(3,3-diphenylpropyl)glycyl]-N-[3-(N,N-dimethylamine)propyl)glycinamide

**3.** An N-alkylglycine trimer according to Claim 1 or 2 that additionally contains a reversible modification with the purpose of increasing its bio-availability and ease of passage of the hematoencephalic barrier and epithelial tissue.

**4.** A composition that comprises at least one formula (I) N-alkylglycine trimer according to any of the Claims 1 to 3.

**5.** A pharmaceutical composition that comprises a therapeutically efficient amount of at least one formula (I) N-alkylglycine trimer according to any of the previous claims 1 to 3, and at least one pharmaceutically acceptable excipient.

**6.** Use of a formula (I) N-alkylglycine trimer according to any of the Claims 1 to 3, in the elaboration of a medicine that inhibits the nervous activity of primary sensorial neurones implicated in the sensations of pain caused by the application of exogenous chemical substances or by heat stimuli or by chemicals endogenously liberated by inflamed tissues.

**7.** Use of a formula (I) N-alkylglycine trimer according to any of the Claims 1 to 3, in the elaboration of a medicine that inhibits the ionic channels activated by exogenous chemical substances or by heat stimuli or by inflammation mediators that lead to the sensation of pain.

**8.** Use of a formula (I) N-alkylglycine trimer according to any of the Claims 1 to 3, in the elaboration of a medicine for the treatment of illnesses and pathological alternations mediated by the ionic channel activity of VR1 nociceptors and/or of the glutamate ionotropic receptors.

**9.** Use of a formula (I) N-alkylglycine trimer according to Claim 8, in the elaboration of a medicine for treatment of pain.

**10.** Use of a formula (I) N-alkylglycine trimer according to Claim 8, in the elaboration of a medicine for the treatment of heat-hyperalgesia.

**11.** Use of a formula (I) N-alkylglycine trimer according to Claim 8, in the elaboration of a medicine for the treatment of neuropathic pain and neurogenic inflammation.

**12.** A cosmetic composition that comprises a cosmetically efficient amount of at least one formula (I) N-alkylglycine trimer according to any of the Claims 1 to 3 and at least one cosmetically acceptable excipient or adjuvant.

**13.** Cosmetic composition according to Claim 12, for skin treatment and care, selected from among an aftersun product, an after-shaving product and an after-depilation product.

**14.** Use of a formula (I) N-alkylglycine trimer according to any of the Claims 1 to 3, in the elaboration of a cosmetic composition, useful for calming, reducing, attenuating or relieving pain or cutaneous irritation produced by heat, mechanical or chemical stimuli or by heat-hyperalgesia.
